(19) Europäisches Patentamt
European Patent Office
Office européen des brevets

(11) Numéro de publication : **0 334 720 B1**

# (12) FASCICULE DE BREVET EUROPEEN

(45) Date de publication du fascicule du brevet : **19.11.92 Bulletin 92/47**

(51) Int. Cl.$^5$ : **C07C 265/16,** C07C 263/02, C07D 213/54, C07D 307/54

(21) Numéro de dépôt : **89400711.1**

(22) Date de dépôt : **15.03.89**

## (54) Nouveau procédé de préparation d'isocyanates d'acyle.

(30) Priorité : **25.03.88 FR 8803915**

(43) Date de publication de la demande : **27.09.89 Bulletin 89/39**

(45) Mention de la délivrance du brevet : **19.11.92 Bulletin 92/47**

(84) Etats contractants désignés : **BE CH DE ES FR GB IT LI NL**

(56) Documents cités :
**DE-C- 275 215**
**US-A- 3 155 700**
**US-A- 4 529 819**

(73) Titulaire : **SOCIETE NATIONALE DES POUDRES ET EXPLOSIFS**
**12, quai Henri IV**
**F-75181 Paris Cédex 04 (FR)**

(72) Inventeur : **Caubere, Paul**
**11, rue de Serre**
**F-54000 Nancy (FR)**
Inventeur : **Den, Min-Zhi Shanghai Institute of Organic Chemistry Academia Sinica**
**345 Lingling Lu Shanghai (CN)**
Inventeur : **Lecolier, Serge**
**3, Allée des Cartelines**
**F-91510 Janville/sur/Juine (FR)**
Inventeur : **Senet, Jean-Pierre**
**79, rue de la Gare Herbeauvilliers-Buthiers**
**F-77760 la Chapelle la Reine (FR)**

(74) Mandataire : **Pech, Bernard et al**
**SNPE - Service Propriété Industrielle 12, Quai Henri IV**
**F-75181 Paris Cédex 04 (FR)**

**EP 0 334 720 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

L'invention concerne un nouveau procédé de préparation d'isocyanates d'acyle à partir d'halogénures d'acyle.

Les isocyanates d'acyle sont des intermédiaires très utiles pour la préparation d'urées, de carbamates et de polymères.

Quelques procédés ont été proposés pour préparer ces isocyanates.

Un des procédés consiste à faire réagir un halogénure d'acyle avec un cyanate d'un métal lourd, tel qu'un cyanate d'argent ou un cyanate de mercure (brevet DE 275 215). Malheureusement le cyanate d'argent est un réactif très onéreux. Lorsqu'on utilise le cyanate de mercure il se forme des sels de mercure qui sont très toxiques et très difficiles à éliminer. Le procédé ne peut, par conséquent, pas être utilisé industriellement.

Selon un autre procédé (brevet US 3 155 700) on fait réagir un halogénure d'acyle avec l'acide isocyanique en présence d'une base faible telle qu'une amine tertiaire.
Mais l'acide isocyanique est très instable (cf. Traité de Chimie Organique V. Grignard XIV (1949) P. 172. Il n'est pas commercialisé et il est très difficile à préparer aussi une utilisation à grande échelle de ce procédé n'est pas possible.

Un autre procédé (brevet US 4 529 819) consiste à faire réagir un chlorure de benzoyle avec un cyanate de métal alcalin en présence de chlorure cuivreux mais les rendements obtenus sont faibles et ne dépassent pas 50 %.

Il existait par conséquent un grand besoin d'un procédé économique de préparation des isocyanates d'acyle.

La présente invention a pour objet un procédé de préparation qui ne présente pas les inconvénients des procédés antérieurs et qui permet d'obtenir de nombreux isocyanates d'acyles avec un très bon rendement, de façon simple et à partir de composés bon marché.

Plus particulièrement la présente invention concerne un procédé de préparation des isocyanates d'acyle de formule

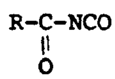

dans laquelle
R représente

- un radical alkyle linéaire ou ramifié en $C_1$ à $C_4$,
- un radical phényle ou naphtyle portant les groupes $R^1$, $R^2$ et $R^3$,

  $R^1$ représentant un atome d'hydrogène, de fluor, chlore ou brome, un radical alkyle en $C_1$ à $C_4$, halogénoalkyle en $C_1$ à $C_4$, alcoxy en $C_1$ à $C_4$ ou fluoro- ou chloroalcoxy en $C_1$ à $C_4$,

  $R^2$ représentant un atome d'hydrogène, de fluor, chlore ou brome, un radical alkyle en $C_1$ à $C_4$, halogénoalkyle en $C_1$ à $C_4$, alkoxy en $C_1$ à $C_4$ ou fluoro- ou chloroalcoxy en $C_1$ à $C_4$,

  $R^3$ représentant un atome d'hydrogène, de fluor, chlore ou brome, un radical alkyle en $C_1$ à $C_4$, halogénoalkyle en $C_1$ à $C_4$, un groupe nitro ou cyano ou représentant un radical aryloxy lorsqu'il n'est pas fixé sur un carbone voisin du carbone lié à la fonction

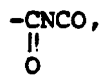

- un radical hétérocyclique aromatique à cinq ou six chaînons, porteur ou non de substituants choisis parmi les radicaux alkyles en $C_1$ à $C_4$ et les atomes de fluor, chlore ou brome, le ou les hétéroatomes étant choisis parmis les atomes d'oxygène, de soufre ou d'azote,

qui consiste à faire réagir un halogénure d'acyle de formule

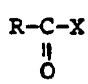

dans laquelle R a la signification précédente et X représente un atome de fluor, chlore ou brome avec le cyanate de sodium ou de potassium, dans un solvant inerte en présence d'au moins un composé choisi

parmi les halogénures d'étain IV ou de zinc,

Dans la présente demande et dans les revendications le terme "halogénoalkyles" représente des radicaux alkyles contenant de 1 à 5 atomes de fluor, chlore ou brome.

La réaction peut être représentée par l'équation suivante :

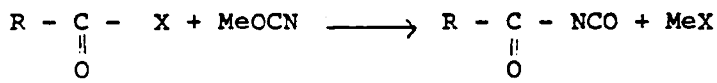

Dans les formules R et X ont les significations précédentes et Me représente le sodium ou le potassium.

Les halogénures d'acyle utilisés comme composés de départ dans le procédé selon l'invention sont des composés que l'on trouve dans le commerce ou qui peuvent être préparés facilement selon des procédés connus (Houben-Weyl, Methoden Der Organischen Chemie (1985) E.5/1 pp. 587 à 609).

pour des raisons économiques on utilise généralement les chlorures d'acyle.

A titre d'exemples d'halogénures d'acyle utilisables comme composés de départ on peut citer

- les halogénures dans lesquels R est un radical méthyle ou éthyle tels que les chlorures d'acétyle et de propionyle,
- les halogénures dans lesquels R est un radical phényle ou naphtyle portant les groupes $R^1$, $R^2$ et $R^3$,

$R^1$ représentant un atome d'hydrogène, de fluor ou de chlore, un radical alkyle en $C_1$ à $C_2$, alcoxy en $C_1$ à $C_2$, le radical $CF_3$, $CCl_3$, $CBr_3$ ou $OCF_3$,

$R^2$ représentant un atome d'hydrogène, de fluor ou de chlore, un radical alkyle en $C_1$ à $C_2$, alcoxy en $C_1$ à $C_2$, le radical $CF_3$, $CCl_3$, $CBr_3$ ou $OCF_3$,

$R^3$ représentant un atome d'hydrogène, de fluor ou de chlore, un radical alkyle en $C_1$ à $C_2$, le radical $CF_3$, $CCl_3$ ou $CBr_3$, ou représentant lorsqu'il n'est pas fixé sur un carbone voisin du carbone lié à la fonction

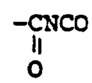

un groupe nitro ou le radical phénoxy ,

tels que les fluorures, chlorures ou bromures d'o-chloro-,m-chloro, p-chloro-, o-méthyl-, m-méthyl-, p-méthyl-, p-trifluorométhyl-, o-méthoxy-, m-méthoxy-, p-méthoxy-, m-nitro-, p-nitro-, 2,6-dichloro-, 2,6-difluoro-, p-phénoxybenzoyle, et

- les halogénures dans lequel R est un radical furyle, thiényle ou pyridyle.

L'halogénure d'acyle est mis à réagir avec le cyanate de sodium ou de potassium de préférence pour des raisons économiques avec le cyanate de sodium. Les quantités sont généralement voisines de la stoechiométrie. On utilise de préférence un léger excès de cyanate.

Il est nécessaire d'effectuer la réaction en présence d'au moins un halogénure d'étain IV ou de zinc. Le terme halogénure représente un fluorure, chlorure, bromure ou iodure.

On a en effet constaté qu'en absence de ces halogénures ou en présence d'autres halogénures métalliques tels que ceux de fer, de titane et d'aluminium les isocyanates d'acyle ne se forment pas.

Les rendements avec les halogénures, en particulier avec les chlorures de zinc sont bons. Ils sont nettement meilleurs avec les halogénures d'étain IV et en particulier avec le chlorure d'étain IV qui est le composé préféré.

La quantité d'halogénure de métal n'est pas critique. Elle ne doit pas être trop faible ni trop élevée si l'on veut obtenir de bons rendements. Elle est en général comprise entre 1 et 10 %, de préférence entre 2 et 6 % en mole par rapport à l'halogénure d'acyle de départ.

Les composés sont dissous ou mis en "suspension dans un milieu solvant de préférence anhydre. Comme solvant on peut utiliser un solvant aromatique halogéné comme le chlorobenzène ou l'o-dichlorobenzène, le trichloroéthylène, le glyme, le diglyme, le dioxane, un mélange d'acétonitrile et d'un solvant non polaire tel que le benzène ou le tétrachlorure de carbone, par exemple dans un rapport 45 : 55.

De préférence, on utilise l'o-dichlorobenzène.

La température de la réaction est généralement comprise entre 75° et 200°C.

De préférence on choisit la température de reflux, en particulier lorsque les composés de départ sont des halogénures d'acyle aromatiques.

La présence d'eau dans le milieu réactionnel pouvant provoquer des réactions secondaires et diminuer le rendement en isocyanates, il est préférable d'effectuer la réaction dans des conditions pratiquement anhydres. Généralement les composés sont mis à réagir sous atmosphère inerte.

Un mode particulier de réalisation de l'invention consiste à faire réagir les composés, par exemple sous azote ou argon, à agiter le milieu réactionnel quelques à la température choisie puis à récupérer l'isocyanate d'acyle après filtration et distillation.

Le présente procédé permet d'obtenir avec un excellent rendement de nombreux isocyanates d'acyle qui peuvent réagir facilement de façon connue sur de nombreux composés, par exemple des amines, des alcools, des phénols pour former des urées et des carbamates particulièrement utiles comme phytosanitaires ou comme médicaments (cf par exemple CA 90:71932 z, 90:152238 e, 91:5028 d, 63:15250 h).

Les exemples qui suivent illustrent l'invention sans toutefois la limiter.

Exemples 1 à 17

Dans ces exemples différents chlorures d'acyle aromatiques ou hétéroaromatiques ont été utilisés.

Mode opératoire général.

Sous atmosphère d'argon et sous agitation on a ajouté le chlorure d'acyle (1 mol) dissous dans de 200 ml d'o-dichlorobenzène déshydraté et $SnCl_4$ à une suspension de NaOCN (1,3 mol) dans 200 ml d'o-dichlorobenzène.

Le mélange a été agité pendant deux heures à la température de reflux (180°C), refroidi à la température ambiante et filtré sous argon. Le filtrat est ensuite distillé sous pression réduite pour obtenir l'isocyanate d'acyle.

Les résultats sont rassemblés dans le tableau I.

TABLEAU I

| EX NUMERO | R | pt. Eb. C° Pa (mm Hg) | RENDEMENT (%) |
|---|---|---|---|
| 1 | 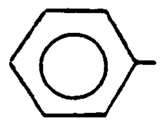 | 74-76<br>1333<br>(10 mm) | 83 |
| 2 | 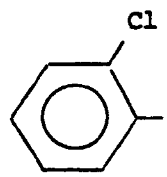 | 98-101<br>267<br>(2 mm) | 80 |
| 3 | 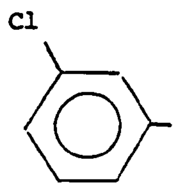 | 103-107<br>666<br>(5 mm) | 81 |
| 4 | 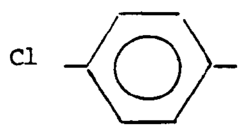 | 92-95<br>267<br>(2 mm) | 85 |
| 5 | 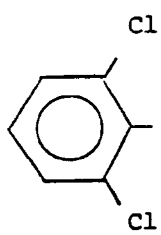 | 123-126<br>133-200<br>(1-1,5 mm) | 87 |
| 6 | 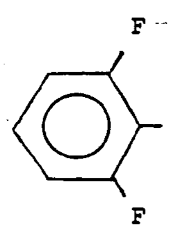 | 83-88<br>1200-1333<br>(9-10 mm) | 70 |

TABLEAU I (Suite)

| EX NUMERO | R | pt. Eb. C° Pa (mm Hg) | RENDEMENT (%) |
|---|---|---|---|
| 7 | 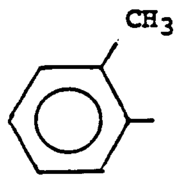 | (183-184)* | (85)** |
| 8 | 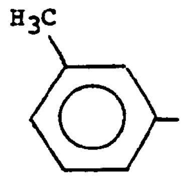 | 84-86<br>267<br>(2 mm) | 80 |
| 9 | 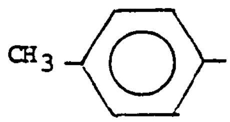 | 82-84<br>213<br>(1,6 mm) | 81 |
| 10 | 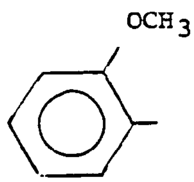 | 115-117<br>133<br>(1 mm) | 67 |
| 11 | 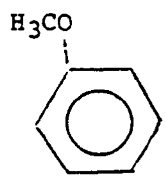 | 104-107<br>267<br>(2 mm) | 78 |
| 12 | 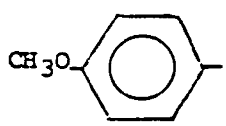 | (200-201)* | (73)** |
| 13 | 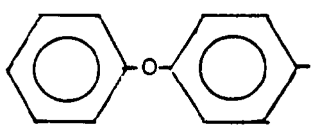 | 131-135<br>66-93<br>(0,5-0,7mm) | 76 |

TABLEAU I (Suite)

| EX NUMERO | R | pt. Eb. C° Pa (mm Hg) | RENDEMENT (%) |
|---|---|---|---|
| 14 | 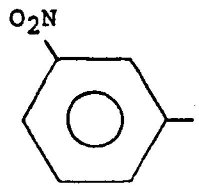 | 103-107 66 (0,5 mm) | 78 |
| 15 | 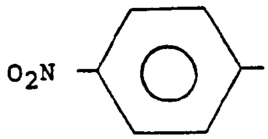 | 120-123 200 (1,5 mm) | 84 |
| 16 | 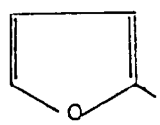 | 80-83 2000 (15 mm) | 71 |
| 17 | 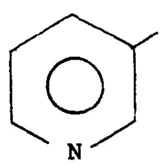 | [1] | 80 |

* Le chiffre entre parenthèses représente le point de fusion de l'urée obtenue par réaction de l'isocyanate formé avec une mole d'aniline.

** Le chiffre entre parenthèses représente le rendement en urée.

[1] Spectre IR : ν (N=C=O) = 2240cm$^{-1}$

Exemple 18

On utilise le même mode opératoire que pour l'exemple 6 mais on remplace le chlorure de difluoro-2,6 benzoyle par le fluorure de difluoro-2, 6 benzoyle. On obtient l'isocyanate de difluoro-2, 6 benzoyle avec un rendement de 75 % . Pt Eb : 78°-80°C sous 66 Pa (0,5 mm Hg).

Exemples 19 et 20

Dans ces exemples les chlorures d'acyle utilisés sont des chlorures d'acyle aliphatiques.

Mode opératoire

Sous atmosphère d'argon et sous agitation on a ajouté le chlorure d'acyle (1 mol) dissous dans 75 ml d'o-dichlorobenzène deshydraté et $SnCl_4$ (0,05 mol) à une suspension de NaOCN (1,3 mol) dans 75 ml d'o-dichlorobenzène deshydraté.

Le mélange a été agité pendant 8 heures à 80° C et filtré sous argon. Le filtrat a été ensuite distillé sous azote pour obtenir l'isocyanate d'acyle. Les résultats sont rassemblés dans le tableau II.

TABLEAU II

| EX NUMERO | R | pt. Eb. (°C) | RENDEMENT (%) |
|---|---|---|---|
| 19 | $CH_3-$ | 78-80 | 36 |
| 20 | $CH_3CH_2-$ | 96-98 | 52 |

Exemple 21

Sous argon et sous agitation on a ajouté 0,2 mole de chlorure de benzoyle dissous dans 100 ml de $CH_3CN$ deshydraté et 125 ml de $C_6H_6$ deshydraté et 0,01 mole de $ZnCl_2$ à une suspension de 0,26 mole de NaOCN dans 100 ml de $CH_3CN$ deshydraté et 125 ml de $C_6H_6$ deshydraté. Le mélange a été agité à la température de reflux (78°C) pendant 8 heures puis refroidi à la température ambiante. Sans isoler l'isocyanate de benzoyle, on a ajouté goutte à goutte au mélange à la température ambiante 0,2 mole de benzylamine dissous dans 100 ml de $C_6H_6$. On a agité pendant une demi-heure puis on a ajouté 1000 ml d'eau pour dissoudre les sels. On a filtré le mélange obtenu et on a récupéré l'urée de formule $C_6H_5CONHCONHCH_2C_6H_5$ sous forme d'un solide blanc qui est recristallisé dans l'éthanol (36,1 g,rendement 71 %), point de fusion 166°C.

Exemples comparatifs 1 à 4

On a utilisé le même mode opératoire que dans l'exemple 21 mais
- dans l'exemple comparatif 1 on n'ajoute aucun halogénure métallique,
- dans l'exemple comparatif 2 on remplace $ZnCl_2$ par $FeCl_3$,
- dans l'exemple comparatif 3 on remplace $ZnCl_2$ par $TiCl_4$,
- dans l'exemple comparatif 4 on remplace $ZnCl_2$ par $AlCl_3$.

Dans tous ces exemples on n'a observé aucune réaction entre le cyanate et le chlorure de benzoyle et on a récupéré ce dernier presque entièrement sous forme de N-benzyl benzamide.

## Revendications

1. Procédé de préparation d'isocyanates d'acyle de formule

$$R-\underset{\underset{O}{\|}}{C}-NCO$$

dans laquelle R représente

- un radical alkyle linéaire ou ramifié en $C_1$ à $C_4$,
- un radical phényle ou naphtyle portant les substituants $R^1$, $R^2$ et $R^3$,

$R^1$ représentant un atome d'hydrogène, de fluor, chlore ou brome, un radical alkyle en $C_1$ à $C_4$, halogénoalkyle en $C_1$ à $C_4$, un radical alcoxy en $C_1$ à $C_4$ ou fluoro- ou chloroalcoxy en $C_1$ à $C_4$,
$R^2$ représentant un atome d'hydrogène, de fluor, chlore ou brome, un radical alkyle en $C_1$ à $C_4$, halogénoalkyle en $C_1$ à $C_4$, un radical alkoxy en $C_1$ à $C_4$ ou fluoro- ou chloroalcoxy en $C_1$ à $C_4$,
$R^3$ représentant un atome d'hydrogène, de fluor, chlore ou brome, un radical alkyle en $C_1$ à $C_4$, halogénoalkyle en $C_1$ à $C_4$, un groupe nitro ou cyano ou représentant un radical aryloxy lorsqu'il n'est pas fixé sur un carbone voisin du carbone lié à la fonction

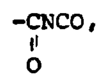

- un radical hétérocyclique aromatique à cinq ou six chaînons, porteur ou non de substituants choisis parmi les radicaux alkyles en $C_1$ à $C_4$ et les atomes de fluor, chlore ou brome, le ou les hétéroatomes étant choisis parmi les atomes d'oxygène, de soufre ou d'azote,

caractérisé en ce que l'on fait réagir un halogénure d'acyle de formule

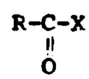

dans laquelle R a la signification précédente et X représente un atome de fluor, chlore ou brome avec une cyanate de sodium ou de potassium, dans un solvant inerte en présence d'au moins un composé choisi parmi les halogénures d'étain IV ou de zinc.

**2.** Procédé selon la revendication 1 caractérisé en ce que X représente le chlore.

**3.** Procédé de préparation selon la revendication 1 ou 2 caractérisé en ce que R représente

- un radical méthyle ou éthyle,
- un radical phényle ou naphtyle portant les groupes $R^1$, $R^2$ et $R^3$,

$R^1$ représentant un atome d'hydrogène, de fluor ou de chlore, un radical alkyle en $C_1$ à $C_2$, alcoxy en $C_1$ à $C_2$, le radical $CF_3$, $CCl_3$, $CBr_3$ ou $OCF_3$,
$R^2$ représentant un atome d'hydrogène, de fluor ou de chlore, un radical alkyle en $C_1$ à $C_2$, ,alcoxy en $C_1$ à $C_2$, le radical $CF_3$, $CCl_3$, $CBr_3$ ou $OCF_3$,
$R^3$ représentant un atome d'hydrogène, de fluor ou de chlore, un radical alkyle en $C_1$ à $C_2$, le radical $CF_3$, $CCl_3$ ou $CBr_3$, ou représentant lorsqu'il n'est pas fixé sur un carbone voisin du carbone relié à la fonction

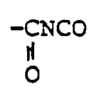

un groupe nitro ou le radical phénoxy,

- un radical furyle, thiényle ou pyridyle.

**4.** Procédé selon l'une quelconque des revendications précédentes, caractérisé en ce qu'on utilise le cyanate de sodium.

**5.** Procédé selon l'une quelconque des revendications précédentes, caractérisé en ce que le solvant est un solvant aromatique halogéné, un mélange acétonitrile-solvant non polaire, le trichloroéthylène, le glyme, le diglyme ou le dioxane.

**6.** Procédé selon la revendication précédente, caractérisé en ce que le solvant est l'o-dichlorobenzène.

**7.** Procédé selon l'une quelconque des revendications précédentes, caractérisé en ce que l'halogénure de

métal est un chlorure.

8. Procédé selon la revendication précédente, caractérisé en ce qu'on utilise le chlorure d'étain IV.

9. Procédé selon l'une quelconque des revendications précédentes, caractérisé en ce que l'halogénure de métal est utilisé en quantité comprise entre 1 et 10 % en mole par rapport à halogénure d'acyle, de préférence entre 2 et 6 % en mole.

10. Procédé selon l'une quelconque des revendications précédentes, caractérisé en ce que le réaction est effectuée à une température comprise entre 75° et 200° C.

## Patentansprüche

1. Verfahren zur Herstellung von Acylisocyanaten der Formel

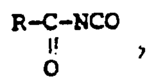

worin R bedeutet

- einen linearen oder verzweigten Alkylrest mit 1-4 Kohlenstoffatomen,
- einen die Reste $R^1$, $R^2$ und $R^3$ als Substituenten enthaltenden Phenyl- oder Naphthylrest, worin $R^1$ ein Wasserstoffatom, ein Fluoratom, ein Chloratom oder ein Bromatom, einen Alkylrest mit 1-4 Kohlenstoffatomen, einen halogenierten Alkylrest mit 1-4 Kohlenstoffatomen, einen Alkoxyrest mit 1-4 Kohlenstoffatomen oder einen fluorierten oder chlorierten Alkoxyrest mit 1-4 Kohlenstoffatomen bedeutet, $R^2$ ein Wasserstoffatom, ein Fluoratom, ein Chloratom oder ein Bromatom, einen Alkylrest mit 1-4 Kohlenstoffatomen, einen halogenierten Alkylrest mit 1-4 Kohlenstoffatomen, einen Alkoxyrest mit 1-4 Kohlenstoffatomen oder einen fluorierten oder chlorierten Alkoxyrest mit 1-4 Kohlenstoffatomen bedeutet, $R^3$ ein Wasserstoffatom, ein Fluoratom, ein Chloratom oder ein Bromatom, einen Alkylrest mit 1-4 Kohlenstoffatomen, einen halogenierten Alkylrest mit 1-4 Kohlenstoffatomen, eine Nitro- oder Cyanogruppe bedeutet oder einen Aryloxyrest, sofern dieser nicht an ein Kohlenstoffatom gebunden ist, das dem an die Gruppe

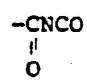

gebundenen benachbart ist,
- einen heterocyclischen aromatischen Rest mit fünf oder sechs Ringatomen, die entweder keine oder solche Substituenten enthalten, die ausgewählt werden unter Alkylresten mit 1-4 Kohlenstoffatomen und Fluoratomen, Chloratomen oder Bromatomen, wobei das oder die Heteroatom(e) ausgewählt werden unter Sauerstoff, Schwefel oder Stickstoff,

dadurch gekennzeichnet,
daß man ein Säurehalogenid der Formel

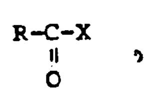

worin R die vorhergehende Bedeutung hat und X ein Fluoratom, ein Chloratom oder ein Bromatom bedeutet, mit Natriumcyanat oder Kaliumcyanat in einem inerten Lösungsmittel in Gegenwart von mindestens einer Verbindung umsetzt, die ausgewählt wird unter den Halogeniden von Sn (IV) oder Zink.

2. Verfahren nach Anspruch 1, dadurch gekennzeichnet, daß X Chlor bedeutet.

3. Verfahren zur Herstellung von Acylisocyanaten nach Anspruch 1 oder 2, dadurch gekennzeichnet, daß R bedeutet:

- einen Methyl- oder Ethylrest,
- einen die Gruppen $R^1$, $R^2$ und $R^3$ enthaltenden Phenyl- oder Naphthylrest, worin $R^1$ ein Wasserstoffatom, ein Fluoratom oder ein Chloratom, einen Alkylrest mit 1-2 Kohlenstoffatomen, einen Alkoxyrest mit 1-2 Kohlenstoffatomen, den Rest $CF_3$, $CCl_3$, $CBr_3$ oder $OCF_3$ bedeutet,
- $R^2$ ein Wasserstoffatom, ein Fluoratom oder ein Chloratom, einen Alkylrest mit 1-2 Kohlenstoffatomen, einen Alkoxyrest mit 1-2 Kohlenstoffatomen, den Rest $CF_3$, $CCl_3$, $CBr_3$ oder $OCF_3$ bedeutet,
- $R^3$ ein Wasserstoffatom, ein Fluoratom oder ein Chloratom, einen Alkylrest mit 1-2 Kohlenstoffatomen, den Rest $CF_3$, $CCl_3$ oder $CBr_3$ bedeutet oder eine Nitrogruppe oder einen Phenoxyrest, wenn er nicht an ein Kohlenstoffatom gebunden ist, das dem an die Gruppe

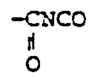

gebundenen benachbart ist,
- oder einen Furylrest, Thienylrest oder Pyridylrest.

**4.** Verfahren nach einem der vorhergehenden Ansprüche, dadurch gekennzeichnet, daß man Natriumcyanat verwendet wird.

**5.** Verfahren nach einem der vorhergehenden Ansprüche, dadurch gekennzeichnet, daß das Lösungsmittel ein halogeniertes aromatisches Lösungsmittel, ein Gemisch aus Acetonitril und einem unpolaren Lösungsmittel, Trichlorethylen, Glyme, Diglyme oder Dioxan ist.

**6.** Verfahren nach Anspruch 5, dadurch gekennzeichnet, daß das Lösungsmittel o-Dichlorbenzol ist.

**7.** Verfahren nach einem der vorhergehenden Ansprüche, dadurch gekennzeichnet, daß das Metallhalogenid ein Chlorid ist.

**8.** Verfahren nach Anspruch 7, dadurch gekennzeichnet, daß man $SnCl_4$ verwendet.

**9.** Verfahren nach einem der vorhergehenden Ansprüche, dadurch gekennzeichnet, daß das Metallhalogenid in einer Menge verwendet wird, die zwischen 1 und 10 Mol-%, bezogen auf das Acylhalogenid, und vorzugsweise zwischen 2 und 6 Mol-% liegt.

**10.** Verfahren nach einem der vorhergehenden Ansprüche, dadurch gekennzeichnet, daß die Reaktion bei einer Temperatur zwischen 75 °C und 200 °C durchgeführt wird.

## Claims

**1.** Process for the preparation of acyl isocyanates of formula

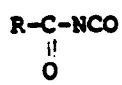

in which R denotes
- a linear or branched $C_1$-$C_4$ alkyl radical,
- a phenyl or naphthyl radical carrying the substituents $R^1$, $R^2$ and $R^3$,
$R^1$ denoting a hydrogen, fluorine, chlorine or bromine atom or a $C_1$-$C_4$ alkyl, $C_1$-$C_4$ haloalkyl radical, $C_1$-$C_4$ alkoxy or $C_1$-$C_4$ fluoro- or chloroalkoxy radical,
$R^2$ denoting a hydrogen, fluorine, chlorine or bromine atom or a $C_1$-$C_4$ alkyl or $C_1$-$C_4$ haloalkyl radical or a $C_1$-$C_4$ alkoxy or $C_1$-$C_4$ fluoro- or chloroalkoxy radical, and
$R^3$ denoting a hydrogen, fluorine, chlorine or bromine atom, or a $C_1$-$C_4$ alkyl or $C_1$-$C_4$ haloalkyl radical, nitro or cyano group or denoting an aryloxy radical when it is not attached to a carbon neighbouring the carbon bonded to the

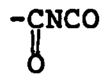

functional group,
- a five- or six-membered aromatic heterocyclic radical carrying or not carrying substituents chosen from $C_1$-$C_4$ alkyl radicals and fluorine, chlorine or bromine atoms, the heteroatom(s) being chosen from oxygen, sulphur or nitrogen atoms,
characterised in that an acyl halide of formula

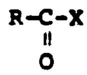

in which R has the above meaning and X denotes a fluorine, chlorine or bromine atom, is reacted with a sodium or potassium cyanate in an inert solvent in the presence of at least one compound chosen from tin(IV) or zinc halides.

2. Process according to Claim 1, characterised in that X denotes chlorine.

3. Process of preparation according to Claim 1 or 2, characterised in that R denotes
   - a methyl or ethyl radical,
   - a phenyl or naphthyl radical carrying the groups $R^1$, $R^2$ and $R^3$,
   $R^1$ denoting a hydrogen, fluorine or chlorine atom, a $C_1$-$C_2$ alkyl or $C_1$-$C_2$ alkoxy radical or the $CF_3$, $CCl_3$, $CBr_3$ or $OCF_3$ radical,
   $R^2$ denoting a hydrogen, fluorine or chlorine atom or a $C_1$-$C_2$ alkyl or $C_1$-$C_2$ alkoxy radical or the $CF_3$, $CCl_3$, $CBr_3$ or $OCF_3$ radical, and
   $R^3$ denoting a hydrogen, fluorine or chlorine atom or a $C_1$-$C_2$ alkyl radical or the $CF_3$, $CCl_3$ or $CBr_3$ radical or, when it is not attached to a carbon neighbouring the carbon bonded to the

   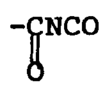

   functional group, a nitro group or the phenoxy radical,
   - a furyl, thienyl or pyridyl radical.

4. Process according to any one of the preceding claims, characterised in that sodium cyanate is employed.

5. Process according to any one of the preceding claims, characterised in that the solvent is a halogenated aromatic solvent, an acetonitrile-nonpolar solvent mixture, trichloroethylene, glyme, diglyme or dioxane.

6. Process according to the preceding claim, characterised in that the solvent is o-dichlorobenzene.

7. Process according to any one of the preceding claims, characterised in that the metal halide is a chloride.

8. Process according to the preceding claim, characterised in that tin(IV) chloride is employed.

9. Process according to any one of the preceding claims, characterised in the metal halide is employed in a quantity of between 1 and 10 mol% relative to the acyl halide, preferably between 2 and 6 mol%.

10. Process according to any one of the preceding claims, characterised in that the reaction is carried out at a temperature of between 75° and 200°C.